# EUROPÄISCHE PATENTSCHRIFT

(11) **EP 4 441 709 B1**
(45) Veröffentlichungstag und Bekanntmachung des Hinweises auf die Patenterteilung: **06.05.2026**
(21) Anmeldenummer: 23701902.1
(22) Anmeldetag: 23.01.2023
(51) Int. Cl.: G06T 7/246, G06T 7/62

(54) **VERFAHREN UND MESSVORRICHTUNG ZUR KORREKTUR EINER POSITION EINES MESSPUNKTES**
METHOD AND MEASURING DEVICE FOR CORRECTING A POSITION OF A MEASUREMENT POINT
PROCÉDÉ ET DISPOSITIF DE MESURE POUR CORRIGER UNE POSITION D'UN POINT DE MESURE

(30) Priorität: 24.01.2022 DE 102022101524
(43) Veröffentlichungstag der Anmeldung: 09.10.2024
(73) Patentinhaber: Karl Storz SE & Co. KG, 78532 Tuttlingen (DE)
(72) Erfinder: HAAG, Simon, 78532 Tuttlingen (DE); KEUSER, Jasmin, 78532 Tuttlingen (DE); BUSCHLE, Lukas, 78532 Tuttlingen (DE)
(86) Internationale Anmeldenummer: PCT/EP2023/051513
(87) Internationale Veröffentlichungsnummer: WO 2023/139252

(56) Entgegenhaltungen:
- DE-B4- 10 100 335
- JP-B2- 5 777 317

## Beschreibung

Die vorliegende Erfindung betrifft ein Verfahren zum Korrigieren einer Position eines Messpunktes in einem Messbild, insbesondere in einem endoskopischen und/oder exoskopischen und/oder mikroskopischen und/oder laryngoskopischen Messbildes, gemäß dem Oberbegriff des unabhängigen Anspruchs 1. Die Erfindung betrifft weiterhin ein Verfahren zum Ausgeben zumindest einer statistischen Kenngröße zu einem beweglichen Messobjekt, das insbesondere in einem endoskopischen und/oder exoskopischen und/oder mikroskopischen und/oder laryngoskopischen Messbild umfasst ist gemäß dem Oberbegriff des Anspruchs 6. Ferner betrifft die Erfindung eine Messvorrichtung mit einem Prozessor, der dazu ausgebildet ist, zumindest eines der erfindungsgemäßen Verfahren auszuführen.

Optische Visualisierungssysteme, wie Mikroskope, Exoskope und Endoskope ermöglichen eine Darstellung einer Szene und/oder eines Arbeitsbereiches, in der bzw. dem feinmotorische Arbeiten und/oder visuelle Überprüfungen durchgeführt werden. Bei medizinischen Eingriffen ist der Arbeitsbereich beispielsweise ein Operationsfeld in einem inneren Bereich, beispielsweis innerhalb eines Thorax oder eines Kopfes, des menschlichen Körpers.

Die Exoskopie beschreibt ein Beobachten und ggf. Beleuchten eines Operationsfeld an einem Patienten und/oder eines Objektfeldes an einem beliebigen Objekt ausgehend von einer Stelle abseits, d. h. außerhalb, eines Körpers des Patienten bzw. abseits des Objektes.

Die Endoskopie beschreibt eine bildgebende Technik, bei der ein Endoskop in einen Hohlraum eingeführt wird. Das Fachpersonal, welches einen solchen Eingriff mit einem Endoskop durchführt, betrachtet das vom Endoskop erfasste Bild auf dem Bildschirm und kann auf Basis dieses Bildes seine Handlungen lenken. Bei medizinischen, beispielsweise minimal-invasiven Eingriffen wird ein Endoskop in den Körper eingeführt, um ein inneres Bild des Körpers zu erfassen und auf dem Bildschirm darzustellen. Aufgrund einer oftmals benötigten, präzisen Arbeitsweise des Fachpersonals ist es wünschenswert, dass ein möglichst genaues, hoch aufgelöstes Bild des Hohlraums und/oder des Arbeitsbereiches vermittelt wird, an dem die Überprüfung und/oder die Operation durchzuführen ist.

Die durch das Endoskop erfassten und auf einem Anzeigegerät dargestellten Bilder sind in der Regel zweidimensional, so dass es selbst dem Fachpersonal aufgrund des Fehlens von Tiefeninformationen nicht möglich ist, Dimensionen und/oder Abmessungen eines betrachteten Objektes in der abgebildeten Szene exakt zu bestimmen.

Um diese Problematik zu lösen und/oder auch Tiefenmessungen in einem Messbild zu ermöglichen, sind jedoch auch dreidimensional arbeitende Stereoendoskope und/oder Stereoexoskope und/oder Mikroskope bekannt, bei denen eine Szene aus zwei verschiedenen Blickwinkeln durch zwei Bilderfassungseinheiten aufgenommen wird. Durch die beiden Bilderfassungseinheiten wird, vorzugsweise pro Zeiteinheit und Blickwinkel synchronisiert, je ein Bild von der zu betrachteten Szene aufgenommen. Hieraus resultieren jeweils sogenannte Stereomessbildpaare mit einem gemeinsamen Zeitstempel. Aus einem solchen Stereomessbildpaar können über Verfahren der Stereorekonstruktion Tiefeninformationen ausgewertet werden.

Im Zuge dieser Stereorekonstruktion wird eine Disparität, d. h. ein horizontaler Pixelversatz, in den jeweiligen Stereobildpaaren durch einen Algorithmus pixelweise, d. h. pro Pixel, bestimmt. Objekte in großer Entfernung weisen bei einer Stereoerfassung eine geringe Disparität, d.h. einen geringen Pixelversatz, innerhalb eines jeweiligen Bildpaares auf. Hingegen weisen Objekte im Vordergrund eine große Disparität auf.

Aus der Disparität kann mit Kenntnis weiterer optischer Parameter der Bilderfassungseinheit(en) eine Art Tiefenkarte zu dem stereorekonstruierten Stereomessbild berechnet werden, die beispielsweise pixelweise eine Tiefeninformation des zu betrachtenden Objektes umfasst. Mithilfe der Tiefeninformationen ist es beispielsweise möglich, ein Abstand zwischen zwei beliebigen Bildpunkten im euklidischen Raum zu bestimmen.

Aus dem Stand der Technik sind bereits Verfahren und Vorrichtungen bekannt, die ein dimensionelles Messen zwischen zwei Messpunkten im euklidischen Raum und somit unter Einbeziehung von Tiefeninformationen der jeweiligen Messpunkte an einem Messobjekt ermöglichen. Hierbei werden von einem Anwender, beispielsweise über eine manuelle Eingabe, zwei Messpunkte an einem Messobjekt in einem Stereomessbild ausgewählt, zwischen denen ein Abstand berechnet werden soll. Hierdurch ist es möglich, dass der Anwender beispielsweise Punkte innerhalb des Stereomessbildes auswählt und ihm im Anschluss daran ein Abstand zwischen diesen Punkten, beispielsweise als überlagerte Darstellung im angezeigten Stereomessbild, ausgegeben wird.

Aus der WO2021/138262 A1 und der EP1965699 B1 ist jeweils ein medizinisches Robotersystem bekannt, bei welchem ganz allgemein mittels "Telestration" eine überlagerte Informationsdarstellung in Messbildern erfolgt. Telestration beschreibt das Kommentieren und Markieren von Bildern im OP durch einen Mentor oder eine andere Person, die die OP extern, d. h. beispielsweise über Livevideo, begleitet. Dabei ist es möglich, Telestrationsgraphiken in einem erfassten Stereobild darzustellen und beispielsweise einem bestimmten Messobjekt in dem Stereobild zuzuordnen.

Aus der WO 2019/213432 A1 ist zudem bekannt, dass ein Nutzer in einem zweidimensional dargestellten Messbild Messpunkte an einem Messobjekt auswählen kann, die im Nachgang unter Zuordnung einer disparitätsbasierten Tiefeninformation in dreidimensionale Messpunkte umgewandelt werden. Durch die Zuordnung ihrer jeweiligen Tiefeninformation ist es möglich, dass der Nutzer derart Messpunkte entlang einer Kontur des Messobjektes bei lediglich zweidimensionaler Darstellung des Messobjektes in Bezug auf dieses setzen kann, um so beispielsweise eine dimensionelle Messung entlang der Kontur zu ermöglichen.

Die DE 10100335 B4 offenbart eine Vorrichtung zur Anzeige einer aus einem Untersuchungsfeld gewonnenen Größe, wobei die Größe anhand von Messdaten einer OCT-Vorrichtung bestimmt wird. Hierbei kann die Position einer Markierung in einer Aufnahme eines Objekts bei Bewegung des Objekts aktualisiert werden.

Aus der JP 5777317 B2 ist ein System zur Vermessung einer Atembewegung in Volumenaufnahmen bekannt, wobei zu der Bewegung Werte wie Minimum, Maximum oder eine Änderungsrate bestimmt und ausgegeben werden können.

Aus dem Stand der Technik ist ferner ein Lösungsansatz bekannt, bei dem der Anwender in Echtzeit während einer Videoaufnahme an einem Messobjekt mit einem Stereoendoskop Messpunkte auswählen kann, um nachfolgend einen Abstand zwischen diesen Messpunkten im endoskopischen Bild als überlagerte Darstellung angezeigt zu bekommen. Um mögliche Bewegungen der Bilderfassungseinheit(en) zu kompensieren, findet eine sensorische Erfassung der Bewegung der Bilderfassungseinheit(en) sowie ein optisches Tracking der ausgewählten Messpunkte in dem Stereomessbild statt. Auf Basis der sensorischen Bewegungsdaten kann eine Nachführung der Messpunkte in dem Messbild erfolgen, durch die eine jeweilige Bewegung der Bilderfassungseinheit(en) kompensiert wird.

Obgleich aus dem Stand der Technik die tiefeninformationsbasierte Auswahl von Messpunkten an einem Messobjekt bekannt ist, sind die Verfahren und Systeme unzureichend, wenn sich das Messobjekt während der Beobachtung bewegt. So kommt es beispielsweise im medizinischen Bereich bei der Erfassung von sich bewegenden Messobjekten, insbesondere Organen, z. B. der Lunge, dem Herzen, dem Darm, etc. dazu, dass sich das Messobjekt während der Auswahl eines Messpunktes hin und her bewegt, wodurch ein zuverlässiges Auswählen und/oder ein Anvisieren mit einem Cursor erschwert wird oder gar unmöglich ist. Dies macht es für den Anwender schwer, einen Messpunkt in Bezug auf das Messobjekt korrekt festzulegen.

Ebenfalls ist der Beobachter und/oder Anwender bei sich, insbesondere rhythmisch und/oder mit einer Frequenz, bewegenden Messobjekten vor die Herausforderung gestellt, dass eine ausmessende Messstrecke zwischen zumindest zwei Messpunkten, die in Bezug auf das Messobjekt durch den Beobachter festgelegt wurden, aufgrund der Bewegung des Messobjektes einer zeitlichen Veränderungen unterliegt. So kann beispielsweise ein Messwert zu der Messtrecke in einer Momentaufnahme ggf. nicht korrekt angezeigt werden, da sich das Messobjekt bereits relativ zu seiner vorherigen Position bewegt hat und somit ein tatsächlicher Abstand in der betrachteten Momentaufnahme entweder kleiner oder größer geworden ist. Somit ist die Messgenauigkeit bei bekannten dimensionellen Messverfahren und Messsystemen unzureichend und/oder eine damit erreichbare Genauigkeit wird vom Anwender regelmäßig überschätzt.

Der vorliegenden Erfindung liegt daher eine Aufgabe zugrunde, die vorstehend genannten Nachteile des Standes der Technik zu überwinden. Insbesondere ist es eine Aufgabe, ein Verfahren und eine Messvorrichtung bereitzustellen, durch die eine Genauigkeit beim Bestimmen einer Position eines Messpunktes an einem sich bewegenden Messobjekt erhöht und/oder für einen Anwender erleichtert wird. Insbesondere ist es auch eine Aufgabe, ein Verfahren und eine Messvorrichtung anzugeben, durch die ein Messabstand und/oder eine Messgenauigkeit bei einer dimensionellen Messung zwischen zwei Messpunkten an einem Messobjekt selbst bei einem sich bewegenden Messobjekt möglichst exakt bestimmt werden kann, sodass dem Anwender eine möglichst einfache, aber dennoch vielsagende Information in einem, insbesondere endoskopischen, Messbild eingeblendet und/oder angezeigt werden kann.

Zumindest eine der vorstehend genannten Aufgaben wird durch ein Verfahren mit den Merkmalen des unabhängigen Anspruchs 1 gelöst. Eine ergänzende Lösung zumindest einer der vorstehend genannten Aufgaben wird durch ein Verfahren mit den Merkmalen des Anspruchs 6 angegeben. Ferner werden die Aufgaben durch eine erfindungsgemäße Messvorrichtung gelöst, die zur Durchführung zumindest eines der erfindungsgemäßen Verfahren ausgebildet ist.

Vorteilhafte Weiterbildungen der Erfindung sind in den abhängigen Ansprüchen angegeben. Es versteht sich, dass Ausführungsbeispiele und Ausführungsformen, die in Bezug auf das Verfahren gemäß Anspruch 1 beschrieben wurden, sich in äquivalenter, wenn auch nicht wortgleicher Form auf das Verfahren nach Anspruch 6 beziehen können, ohne für dieses explizit genannt zu werden. Es versteht sich zudem, dass auch sprachübliche Umformungen und/oder ein sinngemäßes Ersetzen von jeweiligen Begrifflichkeiten im Rahmen der üblichen sprachlichen Praxis, insbesondere das Verwenden von durch die allgemein anerkannte Sprachliteratur gestützten Synonymen, mit von dem vorliegenden Offenbarungsgehalt umfasst sind, ohne in ihrer jeweiligen Ausformulierung explizit erwähnt zu werden.

Gemäß einem ersten Aspekt der Erfindung wird ein Verfahren zum, insbesondere graphischen, Korrigieren einer Position eines Messpunktes in einem Messbild, insbesondere in einem endoskopischen und/oder exoskopischen und/oder mikroskopischen und/oder laryngoskopischen Messbild angegeben. Das Verfahren umfasst zumindest die folgenden Schritte: Erfassen eines ersten Messbildes zumindest eines beweglichen Messobjektes; Bestimmen des zumindest einen Messpunktes in Bezug auf das zumindest eine bewegliche Messobjekt in dem ersten Messbild; Erfassen zumindest eines zeitlich auf das erste Messbild folgenden zweiten Messbildes des zumindest einen beweglichen Messobjektes; Berechnen eines Positionsverschiebungs-vektors zwischen dem zumindest einen Messpunkt in dem ersten Messbild und einem zu dem zumindest einen Messpunkt korrespondierenden Bildpunkt in dem zweiten Messbild; und Korrigieren einer Position des in Bezug auf das erste Messbild festgelegten zumindest einen Messpunktes in dem zweiten Messbild auf Basis des berechneten Positionsverschiebungsvektors.

Besonders bevorzugt handelt es sich bei dem ersten und/oder zweiten Messbild jeweils um ein bereits stereorekonstruiertes Stereomessbild, bei dem vorzugsweise zumindest für jedes Pixel in Bezug auf das zumindest eine bewegliche Messobjekt jeweils eine Tiefeninformation auf Basis einer durchgeführten Stereorekonstruktion verfügbar gemacht wird. Um das erste Stereomessbild zu erfassen, wird vorzugsweise hinsichtlich einer vorbestimmten Zeiteinheit synchronisiert je ein Messbild von dem beweglichen Messobjekt aus zwei unterschiedlichen Blickwinkeln (vorbestimmt durch eine Stereobasis zwischen einer ersten und einer zweiten Bilderfassungseinheit) aufgenommen. Die beiden jeweils zeitlich synchron erfassten Messbilder bilden vorzugsweise ein Stereomessbildpaar mit einem gemeinsamen Zeitstempel. Aus jedem Stereomessbildpaar können pro Zeitstempel über Verfahren der Stereorekonstruktion pixelweise Tiefeninformationen ausgewertet werden. Vorzugsweise wird im Zuge der Stereorekonstruktion eine sogenannte Disparität, d. h. ein horizontaler Pixelversatz, zwischen dem jeweiligen Messbild des jeweiligen Stereobildpaares durch einen Algorithmus pixelweise, d. h. pro Pixel, bestimmt. Objekte in großer Entfernung weisen vorzugsweise bei der Stereoerfassung eine geringe Disparität, d.h. einen geringen Pixelversatz, innerhalb eines jeweiligen Stereobildpaares auf. Hingegen weisen Objekte im Vordergrund eine große Disparität auf. Aus der pixelweise ermittelten Disparität kann mit Kenntnis weiterer optischer Parameter der Bilderfassungseinheit(en) vorzugsweise eine Tiefenkarte zu dem stereorekonstruierten ersten und/oder zweiten Stereomessbild berechnet werden, die vorzugsweise eine pixelweise Tiefeninformation des zu betrachtenden Objektes umfasst. Mithilfe der Tiefeninformationen ist es beispielsweise möglich, ein Abstand zwischen zwei beliebigen Messpunkten innerhalb des ersten und zweiten Stereomessbildes im euklidischen Raum zu bestimmen.

Alternativ kann es sich bei dem ersten und zweiten Messbild auch jeweils um ein Messbild handeln, bei dem, insbesondere pixelweise, Tiefeninformationen, d. h., ein jeweiliger Abstand zu Punkten eines Objekts, vorzugsweise zu jedem Pixel mit einem sogenannten Timeof-Flight Sensor ermittelt werden. Endoskope mit solchen Sensoren sind aus dem Stand der Technik, beispielsweise aus der EP 2 599 433 B1 bekannt.

Alternativ kann es sich bei dem ersten und zweiten Messbild jeweils auch um ein Messbild handeln, bei dem eine jeweilige, insbesondere pixelweise Tiefeninformation basierend auf einer sogenannten Pseudostereoskopie bestimmt wird. Bei dieser Pseudostereoskopie werden die Tiefeninformationen aus einem Stereomessbild gewonnen, das zwei zeitlich beabstandete Einzelbilder bzw. Frames eines Videos einer erfassten Bewegung eines Messobjektes umfasst. Bei dem ersten und zweiten Messbild handelt es sich in diesem Fall also jeweils um ein Stereomessbild. Die Einzelbilder werden dabei vorzugsweise von einer konventionellen (Mono-) Optik aufgenommen, überlappen sich vorzugsweise und können ähnlich zu einem Stereomessbildpaar bzw. ähnlich zu den Messbildern eines ersten und zweiten Stereokanals verwendet werden. Die, insbesondere pixelweisen, Tiefeninformationen zu dem jeweiligen Messbild können über Verfahren der Stereorekonstuktion ermittelt werden.

Alternativ kann es sich bei dem ersten und zweiten Messbild auch um Messbilder handeln, bei denen die, insbesondere pixelweisen, Tiefeninformationen auf Basis von künstlicher Intelligenz ermittelt werden. Hierbei ist es beispielsweise möglich, auf Basis von künstlicher Intelligenz aus 2D-Bildern 3D-Informationen zu ermitteln. Derartige Messbilder können beispielsweise durch eine (Mono-) Optik oder (Mono-) Kamera erfasst werden. Aus den erfassten zweidimensionalen Messbildern kann dann durch Anwendung eines Algorithmus, der beispielsweise ein künstliches neuronales Netz abbildet, auf dreidimensionale Bildinformationen, d.h. auf die Tiefeninformationen, geschlossen werden.

Alternativ kann es sich bei dem ersten und zweiten Messbild jeweils auch um Messbilder handeln, bei denen eine jeweilige, insbesondere pixelweise Tiefeninformation basierend auf Größenverhältnissen vorbekannter Strukturen in der Messszene ermittelt wird. Bei diesen Strukturen kann es sich beispielsweise um einen Teil eines Instruments und/oder um eine Markierung auf einem Instrument handeln, die in dem jeweiligen, erfassten Messbild erkennbar ist, um anhand einer zweidimensionalen Abbildung der Struktur in dem Messbild eine Entfernung der Kamera zu der Struktur zu schätzen. Anhand dieser Schätzung kann vorzugsweise auf die jeweilige Entfernung von weiteren Bildpunkten in dem Messbild geschlossen werden.

Erfindungsgemäß findet die Korrektur der Positionsänderung des zumindest einen Messpunktes vorzugsweise Anwendung, um die Auswahl von Messpunkten in einem Messbild, welches ein sich bewegendes Messobjekt zeigt, für den Anwender zu erleichtern. Die erfindungsgemäße Nachverfolgung einer Positionsänderung zumindest zwischen dem ersten und dem zweiten Messbild erfolgt vorzugsweise durch eine mathematisch-optische Nachverfolgung (engl.: optical tracking).

Das erste und das zweite Messbild sind vorliegend jeweils Einzelbilder einer Vielzahl von zeitlich aufeinanderfolgenden Messbildern, die in Aneinanderreihung eine Videoaufnahme des zumindest einen Messobjektes ergeben. Ein zeitlicher Abstand zwischen den Einzelbildern wird vorzugsweise durch eine vorbestimmte Bilderfassungsfrequenz (engl.: frame rate) bestimmt. Es versteht sich, dass das erste Messbild grundsätzlich ein beliebiges Einzelbild einer solchen Videoaufnahme und/oder Videosequenz sein kann und selbstverständlich nicht ein initial erfasstes Bild sein muss. Es versteht sich auch, dass das zweite Messbild grundsätzlich nicht notwendigerweise in zeitlicher Hinsicht unmittelbar auf das erste Messbild folgen muss, sondern ein oder mehrere Messbilder dazwischen erfasst werden können. Das Korrigieren der Position des in Bezug auf das erste Messbild bestimmten zumindest einen Messpunktes in dem zweiten Messbild auf Basis des berechneten Positionsverschiebungsvektors erfolgt vorzugsweise in Echtzeit, so dass der Anwender diese Positionsverschiebung vorzugsweise visuell nicht erfassen kann und es für ihn so erscheint, als hafte der zumindest eine Messpunkt an dem zumindest einen Messobjekt.

Mit anderen Worten wird der zumindest eine Messpunkt vorzugsweise zwischen aufeinanderfolgenden Einzelbildern (engl.: frames) eines Stereolivevideos, das von dem zumindest einen Messobjekt aufgenommen wird, vorzugsweise fortwährend nachverfolgt. Hierdurch ist es möglich, beispielsweise eine Position eines Cursors, der zum Bestimmen und/oder zum Setzen und/oder zum Auswählen des zumindest einen Messpunktes von einem Anwender zumindest durch das erste Messbild bewegt wird, in dem zweiten Messbild zu korrigieren. Allgemeiner formuliert ist es möglich, die Positionsänderung des zumindest einen Messpunktes in jedem zeitlich auf ein erstes Messbild nachfolgenden zweiten Messbild zu korrigieren. Somit ist es möglich, die Bewegung des zumindest einen Messobjektes durch ein jeweiliges Nachführen des Messpunktes zu kompensieren. Der zumindest eine Messpunkt, beispielsweise dargestellt als ein Cursor, klebt somit aus der Sicht des Anwenders an dem zumindest einen Messobjekt. Wenn der Anwender den Cursor, d. h. den in Bezug auf das Messobjekt zu setzenden Messpunkt, bewegen möchte, wird diese Bewegung erfindungsgemäß mit der berechneten Positionsverschiebung, die aus der Bewegung des Messobjektes resultiert, superpositioniert. Der Anwender verschiebt also mit anderen Worten den Cursor relativ zu dem bewegten Messobjekt und nicht eine Pixelposition des Cursors auf einem Bildschirm.

Als Alternative zu der erfindungsgemäßen Lösung wäre es möglich, den zumindest einen Messpunkt auf einem Standbild auszuwählen. Dies hätte jedoch den Nachteil, dass keine zeitliche Information über den Abstand des Messpunktes von der Bilderfassungsvorrichtung verfügbar wäre. Dies würde dazu führen, dass das Messergebnis in zeitlicher Hinsicht ungenau wäre. Zudem wäre es für den Anwender umständlich, beispielsweise ein Zusatzfenster im Workflow unterzubringen.

Es versteht sich, dass die erfindungsgemäßen Verfahrensschritte nicht zwingend in der aufgeführten Reihenfolge durchgeführt werden müssen, sondern diese Reihenfolge auch verändert werden kann. Es ist zudem möglich, dass ein oder mehrere Zwischenschritte zwischen einem oder mehreren Verfahrensschritten durchgeführt werden können.

In einer bevorzugten Ausführungsform ist das erfindungsgemäße Verfahren gemäß dem ersten Aspekt dadurch gekennzeichnet, dass eine Positionsänderung zwischen dem in Bezug auf das erste Messbild bestimmten zumindest einen Messpunkt und dem zu diesem korrespondierenden Bildpunkt in dem zweiten Messbild auf einer, insbesondere rhythmischen und/oder periodischen und/oder frequenzbasierten, Bewegung des beweglichen Messobjektes basiert. Das erfindungsgemäße Verfahren unterscheidet sich also insbesondere vom Stand der Technik dadurch, dass ein Messpunkt nicht nur auf Basis einer Lage- und/oder Positionsänderung der Messvorrichtung in seiner Position in Bezug auf das Messobjekt korrigiert wird. Anstelle dessen erfolgt eine Positionskorrektur des Messpunktes auf Basis der Bewegung des Messobjektes, so dass dieser an dem Messobjekt in visueller Hinsicht zu haften scheint. Dies war bislang im Stand der Technik nicht möglich. Der Positionsverschiebungsvektor wird also vorzugsweise auf Basis der Bewegung des zumindest einen Messobjektes berechnet. Die rhythmische und/oder periodische und/oder frequenzbasierte Bewegung des beweglichen Messobjektes führt dazu, dass sich das Messobjekt fortlaufend relativ zu der verfahrensmäßig eingesetzten Messvorrichtung, insbesondere relativ zu den beiden Bilderfassungseinheiten, bewegt und dadurch unter anderem fortlaufend seinen Abstand zu der jeweiligen Bilderfassungseinheit ändert. Hierdurch ändern sich auch die Tiefeninformationen zu den einzelnen Bildpunkten in Bezug auf das Messobjekt zwischen zwei Messbildern fortlaufend. Bestimmt ein Anwender einen Messpunkt in Bezug auf das Messobjekt, erfolgt diese Bestimmung grundsätzlich unter Zugrundelegung einer bestimmten Tiefeninformation. Erfindungsgemäß, insbesondere durch die Berechnung des Positionsverschiebungsvektors, ist es nun möglich, die aufgrund der Bewegung des Messobjektes, die in alle drei Raumrichtungen erfolgen kann, sich zeitlich verändernde Abstands- und/oder Tiefeninformation bei der Bestimmung des Messpunktes zu berücksichtigen. Infolgedessen haftet der Messpunkt an dem zumindest einen Messobjekt.

In einer weiteren bevorzugten Ausführungsform ist das erfindungsgemäße Verfahren gemäß dem ersten Aspekt dadurch gekennzeichnet, dass das Berechnen des Positionsverschiebungsvektors auf Basis eines Optical-Flow-Algorithmus und/oder eines elastischen Bildregistrierungsalgorithmus und/oder eines Punktewolkenregistrierungsalgorithmus und/oder eines landmarkerbasierten Trackings erfolgt. Grundsätzlich versteht es sich, dass die hier aufgezählten algorithmischen Berechnungsverfahren nicht einschränkend aufzufassen sind, sondern auch andere und/oder ergänzende Berechnungsverfahren Anwendung finden können, um erfindungsgemäß den Positionsverschiebungsvektor zu berechnen.

In einer weiteren bevorzugten Ausführungsform ist das erfindungsgemäße Verfahren gemäß dem ersten Aspekt dadurch gekennzeichnet, dass das erste und das zweite Messbild jeweils ein Einzelbild einer Videosequenz sind. Vorzugsweise wird das zumindest eine bewegliche Messobjekt, insbesondere durch eine endoskopische und/oder exoskopische und/oder mikroskopische und/oder laryngoskopische Messvorrichtung fortlaufend als Videoaufnahme erfasst. Eine derartige Videoaufnahme von dem zumindest einen beweglichen Messobjekt wird vorzugsweise jeweils von einer ersten und einer zweiten Bilderfassungseinheit gemacht. Die erste Bilderfassungseinheit nimmt das Messobjekt vorzugsweise aus einem ersten Blickwinkel auf. Die zweite Bilderfassungseinheit nimmt das Messobjekt vorzugsweise von einem zweiten Blickwinkel auf, wobei sich der erste Blickwinkel von dem zweiten Blickwinkel unterscheidet. Die erste Bilderfassungseinheit erzeugt zur Videoaufnahme des Messobjektes vorzugsweise eine Vielzahl von einzelnen, zeitlich gemäß einer vorbestimmten Bildaufnahmerate (eng.: framerate, gemessen in frames per second) aufeinanderfolgenden Einzelbildern (Frames). Die zweite Bilderfassungseinheit erzeugt zur Videoaufnahme des Messobjektes vorzugsweise eine Vielzahl von einzelnen, zeitlich gemäß einer vorbestimmten Bildaufnahmerate (eng.: framerate, gemessen in frames per second) aufeinanderfolgenden Einzelbildern (Frames). Die Bilderfassungsrate der ersten Bilderfassungseinheit entspricht vorzugsweise der Bildaufnahmerate der zweiten Bilderfassungseinheit, so dass die beiden Bilderfassungseinheiten zeitlich aufeinander synchronisierte Einzelbilder aufnehmen.

In dem erfindungsgemässen Verfahren gemäß dem ersten Aspekt erfolgt das Bestimmen des zumindest einen Messpunktes in Bezug auf das zumindest eine bewegliche Messobjekt auf Basis einer Benutzereingabe, die durch Bewegen eines Cursors auf einem Display innerhalb des ersten Messbildes getätigt wird. Die Benutzereingabe kann vorzugsweise eine manuelle Benutzereingabe oder eine teilautomatische oder automatische Benutzereingabe umfassen. Beispielsweise kann die Benutzereingabe manuell durch einen Joystick, eine Tastatur, eine Maus und/oder einen Touchscreen erfolgen. Alternativ oder ergänzend kann die Benutzereingabe auch teilweise automatisiert, beispielsweise computerunterstützt sein. Auch eine automatische Benutzereingabe, insbesondere durchgeführt von einem Computer, ist möglich. Das erfindungsgemäße Verfahren ist gemäß dem ersten Aspekt dadurch gekennzeichnet, dass das Korrigieren der Position des zumindest einen Messpunktes eine Überlagerung eines Bewegungsvektor des Cursors zur Bestimmen des Messpunktes mit dem Positionsverschiebungsvektor umfasst. Gemäß diesem Ausführungsbeispiel ist es möglich, dass der Cursor, beispielsweise während einer Live-Videoerfassung des Messobjektes, in dem Messbild zu einem anvisierten Messpunkt bewegt wird. Vorzugsweise wird der Cursor bereits während einer derartigen Bewegung innerhalb des Messbildes auf Basis des Positionsverschiebungsvektors, der vorzugsweise von einer periodischen und/oder frequenzbasierten Bewegung des Messobjektes abhängig ist, fortlaufend korrigiert.

In einer weiteren bevorzugten Ausführungsform ist das erfindungsgemäße Verfahren gemäß dem ersten Aspekt dadurch gekennzeichnet, dass der zumindest eine Messpunkt auf einer Benutzerausgabevorrichtung, vorzugsweise stets und/oder fortlaufend und/oder unterbrechungsfrei, in seiner korrigierten Position dargestellt wird. Dem Anwender wird die Positionskorrektur als solche somit vorzugsweise nicht visualisiert. Der Anwender nimmt in Konsequenz lediglich war, dass der zumindest eine Messpunkt an dem zumindest einen beweglichen Messobjekt haftet, diesem also bei seiner Bewegung stets folgt. Der zumindest eine Messpunkt bewegt sich somit vorzugsweise vor dem Auge des Anwenders mit dem zumindest einen Messobjekt in der (Live-) Videoaufnahme mit hin und her. Hierdurch ist es dem Anwender möglich, den Messpunkt mit einer höheren Genauigkeit in Bezug auf das zumindest eine Messobjekt festzulegen, da er beispielsweise eine gewünschte Stelle, an der der Messpunkt gesetzt werden soll, auch während der Bewegung des Messobjektes nachverfolgen und sich so versichern kann, den Messpunkt in Bezug auf das Messobjekt korrekt platziert zu haben.

Gemäß einer bevorzugten Ausführungsform ist es möglich, dass eine Bewegung der für das erfindungsgemäße Verfahren bevorzugt zum Einsatz kommenden Bilderfassungsvorrichtung kompensiert wird. Hierzu wird unter anderem eine Positionsänderung des zumindest einen Messpunktes zwischen dem ersten und dem zweiten Messbild, die beispielsweise durch die Bewegung der Bilderfassungsvorrichtung und/oder durch eine Bewegung des zumindest einen beweglichen Messobjektes bedingt sein kann, durch Methoden der, insbesondere optischen, Nachverfolgung verfolgt. Vorzugsweise wird bei der optischen Nachverfolgung nicht der zumindest eine Messpunkt verfolgt, sondern das beobachtete Messobjekt als solches. Für eine derartige Verfolgung der Bewegung der Bilderfassungsvorrichtung selbst ist vorzugsweise eine größere Anzahl an erkannten (Mess-) Punkten, z.B. an besonders hervortretenden Strukturen wie Kanten oder kontrastreichen Stellen des Messobjektes bekannt. Es sind vorzugsweise mindestens zwei Messpunkte an dem Messobjekt, besonders bevorzugt eine Vielzahl von Messpunkten bekannt. Hierdurch kann vorzugsweise eine translatorische Bewegung im Raum und/oder eine Rotation der Bilderfassungsvorrichtung aus den verfolgten Messpunkten abgeleitet werden. Es versteht sich, dass auch andere Arten der Nachverfolgung zum Einsatz kommen können. Hierdurch kann vorzugsweise ein modifizierter Positionsverschiebungsvektor berechnet werden, durch den auch eine Bewegung der Bilderfassungsvorrichtung berücksichtigt werden kann. Vorzugsweise weist die Bilderfassungsvorrichtung zumindest einen Positionssensor, beispielsweise einen Gyrosensor, einen GPS-Sensor und/oder einen optischen Sensor, auf, durch den eine Positionsänderung der Bilderfassungsvorrichtung in Form eines Sensorsignals erfasst werden kann. Auch kann die Bilderfassungsvorrichtung ein medizinisches Navigationssystem umfassen, das zu optischen oder elektromagnetischen Erfassung einer Position der Bilderfassungsvorrichtung von außerhalb der Bilderfassungsvorrichtung ausgebildet ist. Die Bilderfassungsvorrichtung kann hierzu beispielsweise einen Tracker oder einen Magnetfeldsensor in Zusammenwirkung mit einer Spule für eine elektromagnetische Erfassung aufweisen. Ein derartiges Sensorsignal wird vorzugsweise bei der Berechnung des modifizierten Positionsverschiebungsvektors mit einbezogen. Hierdurch ist es möglich, die Positionsänderung des zumindest einen Messpunktes zumindest in dem zweiten Messbild zu korrigieren, so dass der zumindest eine Messpunkt in der anwenderbezogenen Darstellung an dem zumindest einen beweglichen Messobjekt zu haften scheint, sich also vorzugsweise gegenüber diesem nicht bewegt. Ein Vorteil der Mitführung und/oder der Positionskorrektur des zumindest einen Messpunktes ist zudem, dass ggf. zu diesem Messpunkt vom Anwender ausgewählte Messwerte (z. B. eine vorzugsweise dreidimensionale Position des Messpunkte in einem vorbestimmten Koordinatensystem) und/oder andere Informationen zeitlich, insbesondere über mehrere Messbilder gemittelt werden können. Somit können Messfehler kompensiert werden.

In einem zweiten Aspekt der Erfindung wird ein Verfahren zum Ausgeben zumindest einer statistischen Kenngröße zu einem beweglichen Messobjekt, das insbesondere in einem endoskopischen und/oder exoskopischen und/oder mikroskopischen Messbild umfasst ist, angegeben. Das erfindungsgemäße Verfahren umfasst zumindest die nachfolgend beschriebenen Schritte. Das Verfahren umfasst ein Bereitstellen einer Vielzahl von zeitlich aufeinanderfolgenden Messbildern des zumindest einen beweglichen Messobjektes durch eine Bilderfassungsvorrichtung, wobei die Vielzahl von Messbildern jeweils, insbesondere Informationen über, zumindest eine in Bezug auf das bewegliche Messobjekt auszugebende Messstrecke umfasst. Die Messstecke wird zwischen einem ersten Messpunkt, der in Bezug auf das zumindest eine bewegliche Messobjekt bestimmt wird, und einem zweiten Messpunkt, der in Bezug auf das zumindest eine bewegliche Messobjekt bestimmt wird, berechnet. Die Messstrecke definiert vorzugsweise einen zu messenden Abstand zwischen dem ersten und dem zweiten Messpunkt. Ferner umfasst das Verfahren ein Ermitteln der zumindest einen statistischen Kenngröße, insbesondere bezüglich der zumindest einen Messtrecke und/oder des Abstandes zwischen dem ersten und dem zweiten Messpunkt, in der Vielzahl von zeitlich aufeinanderfolgenden Messbildern, die zumindest teilweise in Zusammenhang mit einer rhythmischen und/oder periodischen und/oder frequenzbasierten Bewegung des zumindest einen beweglichen Messobjektes steht, durch statistische Auswertung. Das Verfahren umfasst ferner ein Ausgeben der ermittelten, zumindest einen statistischen Kenngröße, durch die die Bewegung des zumindest einen beweglichen Messobjektes zumindest teilweise, vorzugweise in ihrer Gesamtheit, beschrieben wird. Das Ausgeben der zumindest einen statistischen Kenngröße erfolgt vorzugsweise an einen Anwender und/oder Nutzer des Verfahrens in graphischer und/oder textueller und/oder akustischer und/oder taktiler Form. Das erfindungsgemäße Verfahren gemäß dem zweiten Aspekt ist dadurch gekennzeichnet, dass eine Positionsänderung des ersten Messpunktes und/oder des zweiten Messpunktes in der Vielzahl von zeitlich aufeinanderfolgenden Messbildern, die zumindest teilweise durch eine Bewegung des zumindest einen beweglichen Messobjektes hervorgerufen wird, durch das erfindungsgemäße Verfahren gemäß dem ersten Aspekt der Erfindung sowie gemäß dessen bevorzugter Ausführungsformen korrigiert wird. Die beiden Messpunkte, die zur Definition der zumindest einen Messstrecke vorzugsweise durch den Anwender in Bezug auf das zumindest eine Messobjekt bestimmt wurden, können somit durch das erfindungsgemäße Verfahren gemäß dem ersten Aspekt in ihrer Position derart, vorzugsweise in Echtzeit, korrigiert werden, dass zumindest einer der beiden Messpunkte in visueller Hinsicht an dem zumindest einen Messobjekt zu haften scheint. Es ist möglich, dass nur einer der beiden Messpunkte durch das erfindungsgemäße Verfahren in seiner Position gegenüber dem zumindest einen Messobjekt korrigiert wird.

Anstelle der erfindungsgemäßen, statischen Untersuchung der Vielzahl von zeitlich aufeinanderfolgenden Messbilder bzw. der Zeitserie der zumindest einen Messstrecke auf periodische Strukturen, wäre es als Alternativlösung auch möglich, dem Anwender die Messergebnisse eines jeden Messbildes anzuzeigen. Dieser Lösungsansatz hätte allerdings gegenüber der erfindungsgemäßen Lösung den Nachteil, dass sich die angezeigten Messabstände zwischen den einzelnen Messbildern schnell ändern bzw. hin- und herspringen würden. Somit wäre ein exaktes Ablesen der Messwerte durch den Anwender erschwert.

Somit wird zumindest eine der vorstehend genannten, der Erfindung zugrundeliegenden Aufgaben dadurch gelöst, dass vorzugsweise für jede Messstrecke eine Vielzahl von Messergebnissen aus zeitlich aufeinanderfolgenden Messbildern auf Basis von Stereorekonstruktionsverfahren bestimmt wird. Durch die Vielzahl von Messergebnissen kann insbesondere eine Auswirkung einer Bewegung des zumindest einen beweglichen Messobjektes auf die zumindest eine Messstrecke abgebildet werden. Der erste und/oder der zweite Messpunkt sind vorzugsweise in Bezug auf Realpunkte des zumindest einen Messobjektes und ggf. dessen Umgebung festgelegt. Bewegt sich nun zumindest einer dieser Realpunkte durch eine Bewegung des Messobjektes, ändert sich je nach Art und Ausmaß der Bewegung der, insbesondere euklidische, Abstand zwischen den beiden Messpunkten. Der Anwender, kann somit vorzugsweise in Echtzeit, die zumindest zwei Messpunkte auswählen. Durch das erfindungsgemäße Verfahren wird dem Anwender vorzugsweise ein Abstand zwischen den beiden ausgewählten Messpunkten, vorzugsweise als graphische Überlagerung in einem Messbild, besonders bevorzugt als graphische Überlagerung in einer Stereovideoaufnahme des zumindest einen Messobjektes angezeigt.

Bei einem vorliegenden Verfahren kann eine Ermittlung einer Vielzahl von Messergebnissen zu einem zu messenden Abstand zwischen zwei Messpunkten an einem Messobjekt, insbesondere eine zeitliche Serie von Messergebnissen, auf Basis von Stereorekonstruktion von Messbildern erfolgen. Diese Vielzahl von Messergebnissen wird beispielsweise durch eine Mittelwertbildung ausgewertet, um derart einen über die Zeit gemittelten Abstand zwischen zwei Messpunkten zu erhalten.

Erfindungsgemäß wird eine Bewegung des Messobjektes und somit eine zeitliche Veränderung des Abstandes durch Stereorekonstruktion einer Vielzahl von zeitlich aufeinanderfolgenden Messbildern erfasst. Der erfasste zeitliche Verlauf des Abstandes, insbesondere ein zeitlicher Verlauf einer Abstandsänderung, wird erfindungsgemäß derart statistisch ausgewertet, dass zumindest eine statistische Kenngröße, die charakteristisch für die Messobjektbewegung ist, in der Vielzahl von zeitlich aufeinanderfolgenden Messbildern erfasst und dem Benutzer ausgegeben werden kann. Hierdurch kann beispielsweise eine Periodizität, insbesondere eine Frequenz und/oder eine Amplitude, des Messabstands bestimmt werden. Somit ist es erfindungsgemäß möglich, dem Anwender, vorzugsweise neben einem statischen Mittelwert, zumindest eine statistische Kenngröße, beispielsweise einen Minimal- und/oder einen Maximalwert und/oder eine Bewegungsfrequenz, zu der Messstrecke auszugeben. Es ist zu erwähnen, dass es sich bei der zumindest einen statistischen Kenngröße auch um einen in zeitlicher Hinsicht gebildeten Mittelwert handeln kann. Die Erfinder haben außerdem erkannt, dass diese Mittelwertbildung zu einer unzureichenden Messgenauigkeit führen kann. Als Lösung dieses Problems dient nun die statistische Auswertung und Analyse der Zeitserie der zumindest einen Messstrecke. Hierbei werden vorzugsweise statistischen Auswertungsfunktionen auf den zeitlichen Messstreckenverlauf angewandt, durch die eine Bewegung des zumindest einen Messobjektes möglichst exakt beschrieben werden kann. Durch die statistische Auswertung sind somit vorzugsweise auch Änderungen in einer Frequenz und/oder einem Rhythmus und/oder einer Periode der Bewegung nachvollziehbar, so dass dem Benutzer selbst bei komplexen Bewegungsabläufen stets eine genaue Beschreibung der Bewegung ausgegeben werden kann.

In einer bevorzugten Ausführungsform ist das erfindungsgemäße Verfahren gemäß dem zweiten Aspekt dadurch gekennzeichnet, dass die zumindest eine statistische Kenngröße zumindest einen von einer Frequenz, einer Amplitude, einem Minimalwert, einem Maximalwert, einem statistischen Mittelwert, einer Standardabweichung und einem statistischen Fehlerindikator umfasst. Es können auch andere statistische Werte umfasst sein, die in der vorstehenden Aufzählung nicht explizit genannt sind. Es kommt bei der Auswahl der Ausgabe der zumindest einen statistischen Kenngröße vorzugsweise auf die Bewegung des zumindest einen beweglichen Messobjektes an. Bevorzugt werden dem Benutzer mehrere statistische Kenngrößen ausgegeben, so dass die Bewegung des Messobjektes möglichst genau beschrieben wird. Besonders bevorzugt wird durch die statistische Auswertung ein zeitlicher Verlauf der zumindest einen statistischen Kenngröße ermittelt.

In einer bevorzugten Ausführungsform ist das erfindungsgemäße Verfahren gemäß dem zweiten Aspekt dadurch gekennzeichnet, dass zu der zumindest einen statistischen Kenngröße ein mittlerer, resultierender Messfehler ausgegeben wird. Durch die Ausgabe des Messfehlers ist es dem Anwender möglich, zu erkennen, ob eine aktuell durchgeführte Abstandsmessung noch den gewünschten Genauigkeitsanforderungen entspricht oder ob sich beispielsweise eine Bewegung des zumindest einen beweglichen Messobjektes derart verändert hat, dass aktuell eine Abstandsmessung mit der geforderten Genauigkeit nicht erfolgen kann. Die Ausgabe des Messfehlers umfasst für den Anwender eine Warnfunktion, die dem Anwender beispielsweise als akustischer, graphischer bzw. visueller und/oder haptischer Hinweis ausgegeben werden kann.

In einer bevorzugten Ausführungsform ist das erfindungsgemäße Verfahren gemäß dem zweiten Aspekt dadurch gekennzeichnet, dass das erste Messbild und/oder das zweite Messbild und/oder die Vielzahl von Messbildern durch Stereorekonstruktion eines jeweiligen Stereobildpaares auf Basis von optischen und/oder dimensionellen Parametern der Bilderfassungsvorrichtung verarbeitet werden. In dieser Ausführungsform sind das erste und das zweite Messbild vorzugsweise jeweils ein Stereomessbild, das jeweils aus einem Stereomessbildpaar gebildet ist. Dabei umfasst die Verarbeitung vorzugsweise eine, insbesondere pixelweise, Berechnung einer Tiefeninformation zu jedem Bildpunkt in ersten und/oder zweiten Messbild (Stereorekonstruktion), um derart eine dimensionelle Messung zwischen zumindest zwei aus einer Aufnahmeszene ausgewählten Messpunkten zu ermöglichen. Die Stereorekonstruktion umfasst vorzugsweise eine Korrektur von Verzerrungseffekten und/oder eine Transformation in jedem Stereobildpaar (Rektifizierung).

Gemäß einem dritten Aspekt der Erfindung ist eine Messvorrichtung angegeben. Die Messvorrichtung umfasst eine Bilderfassungsvorrichtung mit und zumindest einer ersten und einer von dieser beabstandeten zweiten Bilderfassungseinheit und mindestens eine Auswerteeinrichtung. Es versteht sich, dass die Auswerteeinrichtung außerhalb oder innerhalb der Bilderfassungsvorrichtung angeordnet sein kann und dabei dazu eingerichtet ist, mit der Bilderfassungsvorrichtung zu interagieren. Ferner umfasst die Messvorrichtung einen Prozessor, insbesondere eine Auswerte- und/oder Recheneinheit. Der Prozessor kann vorzugsweise in der Auswerteeinrichtung umfasst oder von dieser separiert angeordnet sein. Der Prozessor ist vorzugsweise dazu eingerichtet, zumindest teilweise die Schritte des Verfahrens gemäß dem ersten Aspekt der Erfindung, auch umfassend dessen Ausführungsformen, auszuführen. Alternativ oder ergänzend ist der Prozessor dazu eingerichtet, zumindest teilweise die Schritte des Verfahrens gemäß dem zweiten Aspekt der Erfindung, auch umfassend dessen Ausführungsformen, auszuführen.

Gemäß dem dritten Aspekt ist der Prozessor vorzugsweise dazu eingerichtet, ein erstes Messbild, vorzugsweise in Form von Messbildinformationen, zumindest eines beweglichen Messobjektes zu erfassen und/oder zu ermitteln. Ferner kann der Prozessor dazu eingerichtet sein, die Rechenleistung bereitzustellen, die in technischer Hinsicht zur Bestimmung des zumindest einen Messpunktes in Bezug auf das zumindest eine bewegliche Messobjekt in dem ersten Messbild in graphischer Hinsicht notwendig ist. Der Prozessor ist vorzugsweise dazu eingerichtet, zumindest ein zeitlich auf das erste Messbild folgendes, zweites Messbild des zumindest einen beweglichen Messobjektes zu verarbeiten. Ferner ist der Prozessor dazu eingerichtet, einen Positionsverschiebungsvektor zwischen dem zumindest einen Messpunkt in dem ersten Messbild und einem zu dem zumindest einen Messpunkt korrespondierenden Bildpunkt in dem zweiten Messbild zu berechnen und eine Position des in Bezug auf das erste Messbild festgelegten zumindest einen Messpunktes in dem zweiten Messbild auf Basis des berechneten Positionsverschiebungsvektors zu korrigieren.

Alternativ oder ergänzend kann gemäß dem dritten Aspekt der Prozessor vorzugsweise dazu eingerichtet sein, eine Vielzahl von zeitlich aufeinanderfolgenden Messbildern des zumindest einen beweglichen Messobjektes bereitzustellen. Die Bilderfassungsvorrichtung ist vorzugsweise dazu eingerichtet, eine Vielzahl von zeitlich aufeinanderfolgenden Stereobildpaaren in Form von Stereobildpaarinformationen an den Prozessor zu übermitteln. Der Prozessor ist vorzugsweise dazu eingerichtet, aus jedem Messbildpaar durch Stereorekonstruktion jeweils ein Messbild zu erzeugen bzw. bereitzustellen. Die Vielzahl von Messbildern umfassen zumindest eine in Bezug auf das bewegliche Messobjekt auszugebende Messstrecke, die zwischen einem ersten Messpunkt, der in Bezug auf das zumindest eine bewegliche Messobjekt bestimmt ist, und einem zweiten Messpunkt, der in Bezug auf das zumindest eine bewegliche Messobjekt bestimmt ist, bestimmt wird. Der Prozessor ist vorzugsweise dazu ausgebildet, einen Abstand der zumindest einen Messstrecke zu berechnen. Ferner ist der Prozessor dazu eingerichtet, zumindest einer statistischen Kenngröße in der Vielzahl von zeitlich aufeinanderfolgenden Messbildern, die zumindest teilweise in Zusammenhang mit einer periodischen und/oder frequenzbasierten Bewegung des zumindest einen beweglichen Messobjektes steht, durch statistische Auswertung zu ermitteln. Der Prozessor ist vorzugsweise dazu eingerichtet, die zumindest eine statistische Kenngröße bzw. einen zeitlichen Verlauf der zumindest einen statistischen Kenngröße an die Benutzerausgabevorrichtung zu übermitteln. Die Benutzerausgabevorrichtung ist dazu eingerichtet, die zumindest eine statistische Kenngröße, durch die die Bewegung des zumindest einen beweglichen Messobjektes in zeitlicher Hinsicht zumindest teilweise beschrieben wird, auszugeben.

In einer bevorzugten Ausführungsform umfasst die Messvorrichtung ein Stereoendoskop und/oder ein Stereoexoskop und/oder ein Stereomikroskop und/oder ein Laryngoskop. Vorzugsweise ist die Messvorrichtung als Stereoendoskop und/oder als Stereoexoskop und/oder als Stereomikroskop und/oder als Laryngoskop ausgebildet.

In einer bevorzugten Ausführungsform umfasst die Messvorrichtung eine Benutzereingabevorrichtung und/oder eine Benutzerausgabevorrichtung. Die Benutzereingabevorrichtung kann eine Tastatur und/oder eine Maus und/oder einen Joystick und/oder einen Touchscreen und/oder ein Touchpad und/oder eine sonstige manuelle Eingabevorrichtung umfassen. Die Benutzerausgabevorrichtung kann einen Bildschirm und/oder eine Brille und/oder ein 3D-Brille und/oder eine augmented reality Brille umfassen.

Weitere Vorteile und Einzelheiten der Erfindung ergeben sich aus der nachfolgenden Beschreibung bevorzugter Ausführungsformen der Erfindung sowie anhand von lediglich schematischen Zeichnungen.

Es zeigen:
- FIG 1:: eine schematische Ansicht eines Ausführungsbeispiels einer erfindungsgemäßen Messvorrichtung;
- FIG 2:: ein beispielhaftes Stereomessbild, in dem ein beispielhaftes bewegliches Messobjekt in einer ersten Position erfasst ist, mit einem in Bezug auf das Messobjekt dargestellten Messabstand;
- FIG 3:: ein beispielhaftes Stereomessbild, in dem ein beispielhaftes bewegliches Messobjekt in einer zweiten Position erfasst ist, mit einem in Bezug auf das Messobjekt dargestellten Messabstand;
- FIG 4:: eine graphische Darstellung eines zeitlichen Verlaufes eines Messabstandes;
- FIG 5:: der in FIG. 4 gezeigte graphische Verlauf des Messabstandes mit einer überlagerten Darstellung eines zeitlichen Verlaufes eines Mittelwertes des Messabstandes;
- FIG 6:: ein beispielhaftes Frequenzdiagram, das durch eine statistische Auswertung erfasst ist;
- FIG 7:: der in FIG. 4 gezeigte graphische Verlauf des Messabstandes mit einer überlagerten Darstellung von statistisch berechneten Minima und Maxima.
- FIG 8:: eine beispielhafte Pfeil-Darstellung eines Cursors zur beispielhaften Darstellung eines Positionsverschiebungsvektors und eines Bewegungsvektors des Cursors, der aufgrund einer Benutzereingabe auftritt, zwischen aufeinanderfolgenden Stereomessbildern.

Gleiche Elemente beziehungsweise Elemente mit gleicher Funktion sind in den Figuren mit den gleichen Bezugsziffern versehen.

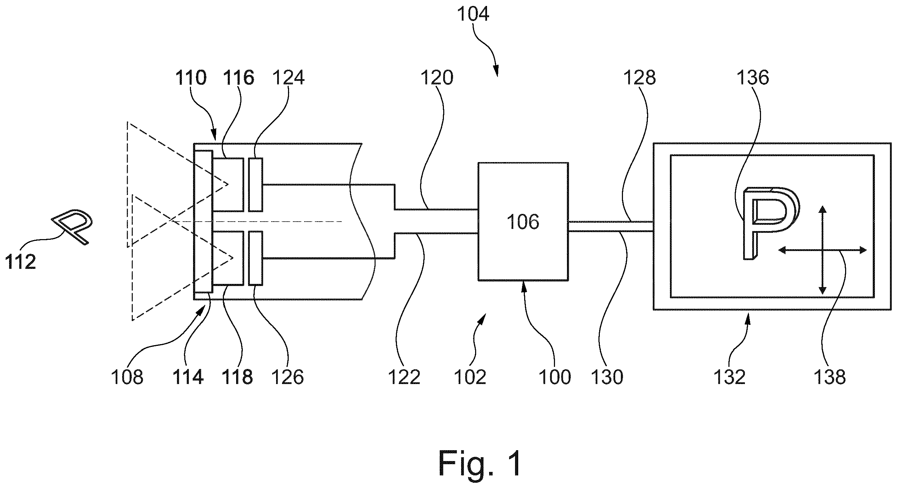

Fig. 1 zeigt in einer schematisch stark vereinfachten Blockdarstellung eine beispielhafte Ausgestaltung einer Messvorrichtung 100. Die Messvorrichtung 100 ist vorliegend in einem Stereo-Endoskop 102 ausgeführt. Die Messvorrichtung 100 weist eine Bilderfassungsvorrichtung 104 mit einer Auswerteeinrichtung 106 auf. Ferner umfasst die Bilderfassungsvorrichtung 104 eine erste Bilderfassungseinheit 108 und eine zweite Bildererfassungseinheit 110.

Die erste Bilderfassungseinheit 108 weist einen vorbestimmten Abstand von der zweiten Bilderfassungseinheit 110 auf, der eine Stereobasis der Stereo-Endoskops definiert. Bei der ersten und der zweiten Bilderfassungseinheit 108, 110 handelt es sich vorzugsweise jeweils um eine Kamera. Die Auswerteeinrichtung 106 ist vorzugsweise dazu eingerichtet, von der ersten und der zweiten Bilderfassungseinheit 108, 110 Bilddaten in Form von Messbildern zu empfangen und diese auszuwerten. Besonders bevorzugt weist die Auswerteeinrichtung 106 zumindest einen nicht näher gezeigten Prozessor zur Bildverarbeitung auf. Es versteht sich, dass die Auswerteeinrichtung 106 in anderen Ausführungen vorzugsweise außerhalb der Bilderfassungsvorrichtung 104 angeordnet sein kann. Die Auswerteeinrichtung 106 ist vorzugsweise als eine sogenannten Kamerakontrolleinheit (Camera Control Unit, CCU) ausgebildet. Vorzugsweise kann zumindest eine Vorverarbeitung der erfassten Messbilder 109, 111 in der Bilderfassungsvorrichtung 104 stattfinden.

Der ersten und der zweiten Bilderfassungseinheit 108, 110 ist eine Objektivbaugruppe 114 zugeordnet. Die Objektivbaugruppe 114 umfasst beispielhaft ein Deckglas und optische Einheiten 116, 118 mit Aperturen, die den Bilderfassungseinheiten 108, 110 zugeordnet sind. Die optischen Einheiten 116, 118 definieren das jeweilige Sichtfeld der Bilderfassungseinheiten 108, 110. Jede der beiden Bilderfassungseinheiten 108, 110 ist einem Beobachtungskanal 120, 122 zugeordnet. Die Beobachtungskanäle 120, 122 sind jeweils dazu ausgebildet, die Messbilder in Form von signalförmigen Bildinformationen an die Auswerteeinrichtung 106 bzw. an den zumindest einen Prozessor zu übermitteln. Zur Bereitstellung der Bildinformationen ist jeder der Bilderfassungseinheiten 108, 110 ein Signalwandler 124, 126 zugeordnet. Die Signalwandler 124, 126 sind jeweils dazu eingerichtet, die optisch erfassten Messbilder in Bildinformationen umzuwandeln. Beispielhaft handelt es sich bei den Signalwandlern 124, 126 um Fotochips.

Die erste Bilderfassungseinheit 108 ist dazu eingerichtet, zumindest ein erstes Messbild von zumindest einem beweglichen Messobjekt 112 zu erfassen. Das Messobjekt 112 ist hier beispielhaft als ein Buchstabe P dargestellt. Allerdings handelt es sich bei dem Messobjekt 112 normalerweise vorzugsweise um ein menschliches oder tierisches Organ oder einen anderen Teil eines menschlichen oder tierischen Körpers oder um ein Bauteil. Die erste Bilderfassungseinheit erfasst das zumindest eine Messbild von dem Messobjekt 112 vorzugsweise aus einem ersten Blickwinkel.

Die zweite Bilderfassungseinheit 110 ist dazu eingerichtet, zumindest ein zweites Messbild des zumindest einen beweglichen Messobjektes 112 zu erfassen. Die ersten und die zweite Bilderfassungseinheit 108, 110 sind jeweils dazu eingerichtet, das erste und das zweite Messbild vorzugsweise zeitlich synchronisiert zu erfassen. Ein derartig erfasstes erstes und zweites Messbild bildet vorzugsweise ein Stereobildpaar aus.

Die Auswerteeinrichtung 106 ist dazu ausgebildet, aus dem Stereobildpaar bzw. aus den signalbasierten Bildinformationen des ersten und zweiten Messbildes durch bekannte Methoden der Stereorekonstruktion Stereomessbildinformationen zu ermitteln. In den Stereomessbildinformationen sind zu jedem erfassten Bildpunkt des zumindest einen Messobjektes 112 Tiefeninformationen verfügbar, die beispielsweise dazu verwendet werden können, einen Abstand zwischen zwei Messpunkten an dem Messobjekt 112 im euklidischen Raum zu berechnen.

Die Stereomessbildinformationen können vorzugsweise über einen ersten und/oder einen zweiten Ausgabekanal 128, 130 an eine Benutzerausgabevorrichtung 132 übertragen werden, durch die die Stereomessbildinformationen einem Benutzer als ein erstes (Stereo-)Messbild 134 bereitgestellt und vorzugsweise graphisch angezeigt werden. Die Benutzerausgabevorrichtung 132 kann beispielsweise ein Display sein. Auf der Benutzerausgabevorrichtung 132 wird das zumindest eine Messobjekt 112 in Form eines Beobachtungsobjektes 136 abgebildet. Ein Benutzer kann mithilfe einer Benutzereingabevorrichtung (nicht gezeigt) vorzugsweise einen Cursor 138 relativ zu dem Beobachtungsobjekt 136 bzw. zu dem virtualisierten Messobjekt 112 verschieben, um beispielsweise einen Messpunkt in Bezug auf das Messobjekt zu bestimmen.

Es versteht sich, dass die Messvorrichtung 100 dazu eingerichtet ist, grundsätzlich eine Vielzahl von Messbildern des Messobjektes zu erfassen und somit eine Vielzahl von Stereomessbildern zu jedem Stereobildpaar zu erfassen bzw. bereitzustellen. Besonders bevorzugt ist es mit der Messvorrichtung 100 möglich, ein (Live-) Video von dem zumindest einen Messobjekt 112 aufzunehmen, dass aus einer Vielzahl von Einzelbildpaaren zusammengesetzt ist, die in einem vordefinierten zeitlichen Abstand (bestimmt durch die frame rate) hintereinander erfasst werden.

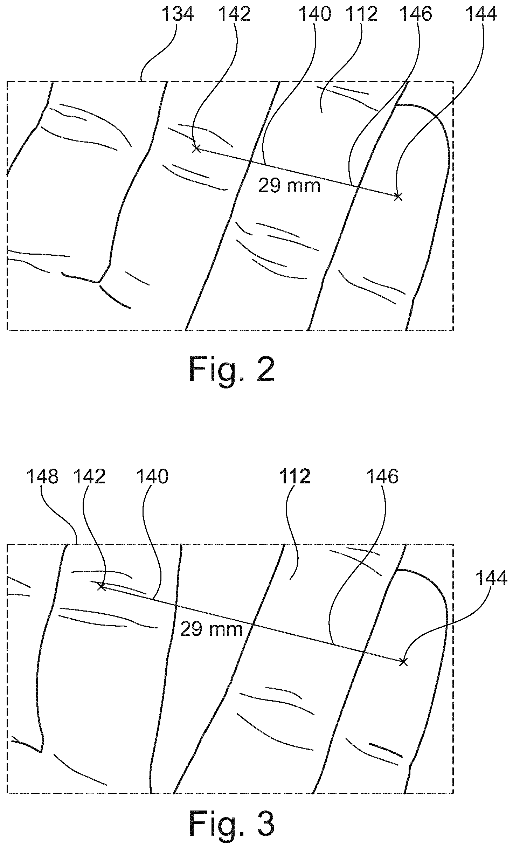

In Fig. 2 ist beispielhaft ein erstes Stereomessbild 134 vereinfacht im Zweidimensionalen dargestellt, das beispielsweise von der erfindungsgemäßen Messvorrichtung 100 aufgenommen wurde. Es ist ersichtlich, dass es sich bei dem Messobjekt nunmehr nicht mehr um das in Fig. 1 beispielhaft gezeigte Messobjekt handelt, sondern ein menschliches Körperteil als Messobjekt 112 durch das erste Stereomessbild 134 dargestellt ist. Das zumindest eine Messobjekt 112 wird vorliegend durch Finger einer Hand repräsentiert. Das zumindest eine Messobjekt 112 kann jedoch auch jegliche andere Art von Messobjekt, beispielsweise einen Teil einer Anatomie und/oder ein oder mehrere Organe des menschlichen oder tierischen Körpers umfassen. Alternativ kann es sich bei dem zumindest einen Messobjekt 112 auch um ein Bauteil handeln. Gemäß Fig. 2 ist in dem ersten Stereomessbild 134 beispielhaft ein Abstand 140 zwischen einem ersten Messpunkt 142 und einem zweiten Messpunkt 144 dargestellt, der dem Benutzer bzw. Anwender in visueller Hinsicht sowohl als Messstrecke 146 und zusätzlich als Zahlenwert, im Beispiel 29 mm, als mit dem ersten Stereomessbild 134 überlagerte Graphikelemente angezeigt wird.

Der erste Messpunkt 142 wird von einem Anwender vorzugsweise durch den Cursor 138, der auf der Benutzerausgabevorrichtung 132 und/oder in einer Brille des Anwenders angezeigt ist, in Bezug auf das zumindest eine bewegliche Messobjekt 112 beispielhaft in dem ersten Stereomessbild 134 bestimmt und/oder festgelegt. Der zweite Messpunkt 144 wird von einem Anwender vorzugsweise durch den Cursor 138, der auf der Benutzerausgabevorrichtung 132 und/oder in einer Brille des Anwenders angezeigt ist, in Bezug auf das zumindest einen bewegliche Messobjekt 112 beispielhaft in dem ersten Stereomessbild 134 bestimmt und/oder festgelegt.

In Fig. 3 ist beispielhaft ein in zeitlicher Hinsicht auf das erste Stereomessbild 134 folgendes zweites (Stereo-)Messbild 148 dargestellt. Das zumindest eine Messobjekt 112 befindet sich gemäß dem zweiten Stereomessbild 148 in einer zweiten Position. Die Positionsänderung des zumindest einen Messobjektes 112 ist auf eine Bewegung des Messobjektes 112, im vorliegenden Beispielfall auf ein rhythmisches und/oder frequentes Öffnen und Schließen der Finger der Hand zurückzuführen. Das erste und das zweite Stereomessbild 134, 148 stellen jeweils eine beispielhafte Momentaufnahme dieser rhythmischen bzw. frequenten Bewegung dar.

Das erste und das zweite Stereomessbild 134, 148 sind vorliegend jeweils Einzelbilder einer Vielzahl von zeitlich aufeinanderfolgenden Messbildern (vgl. Ausführungen zur Fig. 1), die in Aneinanderreihung eine Videoaufnahme des zumindest einen Messobjektes 112 ergeben. Jedes Stereomessbild der Vielzahl von Stereomessbildern, also auch das erste und das zweite Stereomessbild 134, 148 resultiert aus jeweils einem Stereobildpaar, das durch bekannte Verfahren der Stereorekonstuktion rekonstruiert wird, so dass vorzugsweise für jeden erfassten Bildpunkt des zumindest einen Messobjektes 112 eine Tiefeninformation verfügbar ist. Aus jedem Stereobildpaar und/oder aus dem bereits stereorekonstruierten Stereomessbild wird der Abstand 140 zwischen dem ersten und den zweiten Messpunkt 142, 144 im euklidischen Raum berechnet.

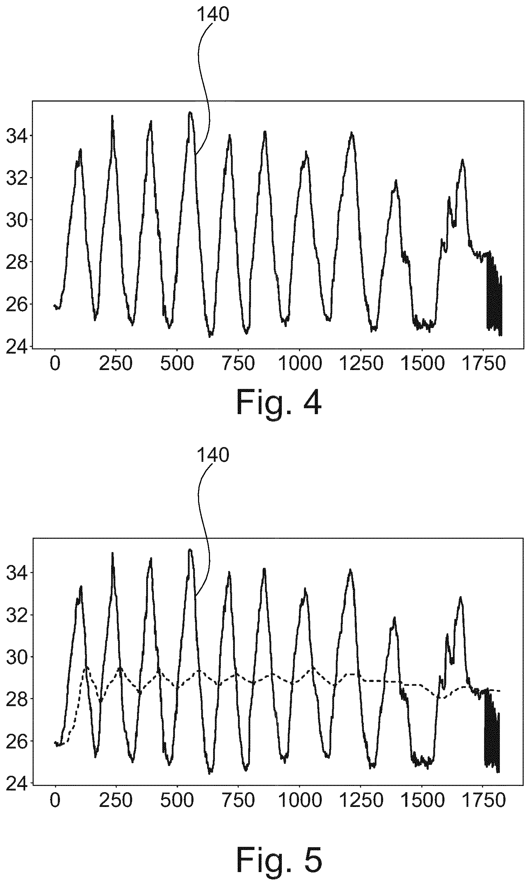

Hierdurch ergibt sich eine Zeitserie von Messstrecken 146, die beispielhaft in dem in Fig. 4 gezeigten Diagramm dargestellt ist. Auf der Abszisse ist die Bildfolgennummer (engl.: frame number) angezeigt. Auf der Ordinate ist der sich verändernde Abstand in Millimetern der sich öffnenden und schließenden Finger dargestellt. Die Abstandsänderung zwischen zwei Messstellen an dem beweglichen Messobjekt 112, die in visueller Hinsicht durch die beiden Messpunkten 142, 144 markiert werden, kann unabhängig von der optischen bzw. visualisierten Nachführung der Messpunkte 142, 144 (deren Anhaftung an dem Messobjekt) durch Verfahren zu Bestimmung des optischen Flusses sowie durch ähnliche Verfahren berechnet werden.

Wie aus einer Zusammenschau der Figuren 2 und 3 zu entnehmen ist, wird sowohl der erste als auch der zweite Messpunkt 142, 144 hinsichtlich seiner Position in Abhängigkeit der Bewegung des zumindest einen Messobjektes 112 gemäß dem erfindungsgemäßen Verfahren korrigiert und haftet derart in visueller Hinsicht in der Ansicht, die dem Anwender der Messvorrichtung 100 angezeigt wird, an dem Messobjekt 112. Hierfür wurde die open-source-Funktion von openCV cv2.calcOpticalFlowPyrLK() verwendet. Grundsätzlich sind jedoch auch andere Optical-Flow Berechnungsverfahren und/oder ein Landmarken-basiertes Tracking zur Positionsnachverfolgung und Positionskorrektur der beiden Messpunkte 142, 144 möglich.

Gemäß dem in den Fig. 2 und 3 gezeigten Ausführungsbeispiel wird die dem Anwender angezeigte Messstrecke 146 allerdings nicht nach dem ebenfalls erfindungsgemäßen Verfahren in statistischer Hinsicht ausgewertet. Der Abstand 140 zwischen den zwei Messpunkten 142, 144, wird vielmehr lediglich zeitlich gemittelt. Diese zeitliche Mittelwertbild führt, wie in Fig. 3 dargestellt ist, dazu, dass dem Anwender in dem überlagerten Graphikelement ein falscher Zahlenwert, vorliegend beispielhaft 29mm, für den aktuell in dem zweiten Stereomessbild 148 dargestellten Abstand 140 angezeigt wird. Diese fehlerhafte Darstellung kann durch das erfindungsgemäße Verfahren gemäß dem zweiten Aspekt grundlegend verbessert werden.

In Übereinstimmung mit der fehlerbehafteten Darstellung des Abstandes 140 in dem in Fig. 3 gezeigten zweiten Stereomessbild 148 ist in Fig. 5 die aus Fig. 4 bekannte Abstands-Bildfolgennummer zusammen mit einer zu diesem berechneten Mittelwertkurve dargestellt. Es ist ersichtlich, dass sich im zeitlichen Mittel ein Abstand 140 mit einem Mittelwert von ca. 29 mm ergibt. Durch die zeitliche Mittelung des Abstandes 140 hinkt der angezeigte Zahlenwert, wie in Fig. 3 beispielhaft dargestellt, in zeitlicher Hinsicht jedoch hinterher, was zu einer Unsicherheit über die Korrektheit der Anzeige führen kann. Der statistische Fehler dieser Messung ist versuchsseitig mit 3 mm anzugeben und daher im Vergleich zu der erfindungsgemäß ermöglichten Auswertung ungenau.

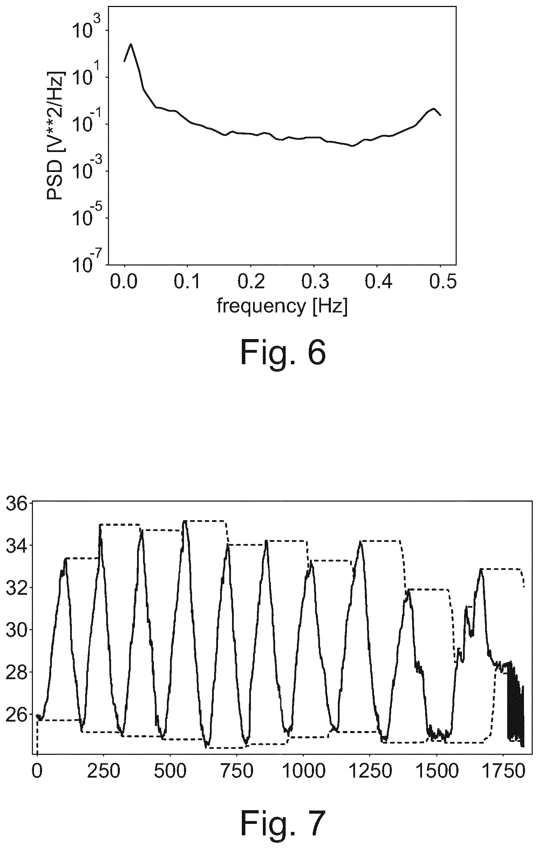

Anstelle der in Fig. 5 beispielhaft gezeigten Mittelwertbildung, wird die ermittelte Zeitserie der Messstrecke 146 erfindungsgemäß zusätzlich mittels statistischer Methoden, beispielsweise durch Berechnung einer Periodizität und/oder einer Amplitude, analysiert. Die Periodizität und/oder eine Amplitude repräsentieren beispielhaft die zumindest eine statistische Kenngröße. Beispielhaft ist als Resultat einer derartigen Analyse in Fig. 6 ein Periodigramm (max. bei 0,006 1/frames entspricht 170 frames) der Frequenzanalyse dargestellt. Zudem wurde beispielhaft eine Amplitude (angezeigt als Minima und Maxima) der ermittelten Zeitserie der Messstrecke 146 ermittelt und ist in Fig. 7 angezeigt. Durch die Berechnung dieser statistischen Parameter können dem Anwender Minimal - und Maximalwerte sowie ggf. Frequenzen einer Bewegung des zumindest einen Messobjektes, vorzugsweise graphisch, angezeigt werden.

Besonders bevorzugt kann aus der in statistischer Hinsicht analysierten Bewegung des zumindest einen Messobjektes 112 zumindest ein Vitalparameter, der für das zumindest eine Messobjekt signifikant ist, ermittelt werden. Handelt es sich bei dem zumindest einen Messobjekt 112 beispielsweise um ein Herz eines Menschen oder eines Tieres, ist es durch das erfindungsgemäße Verfahren möglich, auf Basis der zumindest einen statistischen Kenngröße eine Atemfrequenz und/oder eine Herzschlagfrequenz zu ermitteln. Umfasst das zumindest einen Messobjekt 112 zumindest eine objektoberflächlich angeordnete Vene und/oder Arterie, ist es möglich, durch das erfindungsgemäße Verfahren auf Basis der zumindest einen statistischen Kenngröße einen Herzschlag zu ermitteln.

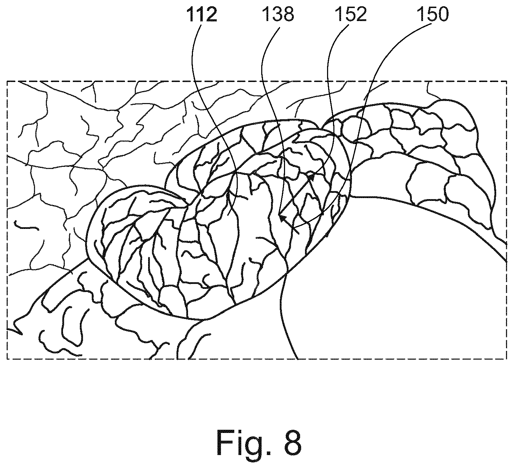

In Fig. 8 ist ein Anwendungsbeispiel des erfindungsgemäßen Verfahrens gemäß dem ersten Aspekt dargestellt. Zur Bestimmung der Position des Cursors 138 zur Auswahl von Messpunkten 142, 144 werden zwei verschiedene Verschiebungsvektoren berechnet. Einerseits wird aus einem optischen Tracking wie oben beschrieben ein Positionsverschiebungsvektor 150 zwischen zwei aufeinanderfolgenden Stereomessbilder bestimmt. Zudem wird ein Bewegungsvektor 152 aus einer, vorzugsweise manuellen, Benutzereingabe, z.B. durch einen Joystick, eine Tastatur oder eine Maus, bestimmt. Die beiden Vektoren 150, 152 werden superponiert und der Cursor 138 kann entsprechend verschoben werden. Dies führt dazu, dass sich eine Cursorposition nur relativ zu dem zumindest einen Messobjekt 112 ändert. Der Cursor 138 bzw. Messpunkt haftet somit vorzugsweise an dem Messobjekt, solange der Benutzer keine andere Bewegung vorgibt oder eine andere Eingabe ausführt.

### Bezugszeichenliste

- 100: Messvorrichtung
- 102: Stereo-Endoskop
- 104: Bilderfassungsvorrichtung
- 106: Auswerteeinrichtung
- 108: erste Bilderfassungseinheit
- 110: zweite Bilderfassungseinheit
- 112: Messobjekt
- 114: Objektivbaugruppe
- 116: optische Einheit
- 118: optische Einheit
- 120: Beobachtungskanal
- 122: Beobachtungskanal
- 124: Signalwandler
- 126: Signalwandler
- 128: erster Ausgabekanal
- 130: zweiter Ausgabekanal
- 132: Benutzerausgabevorrichtung
- 134: erstes Messbild
- 136: Beobachtungsobjekt
- 138: Cursor
- 140: Abstand
- 142: erster Messpunkt
- 144: zweiter Messpunkt
- 146: Messstrecke
- 148: zweites Messbild
- 150: Positionsverschiebungsvektor
- 152: Bewegungsvektor

## Patentansprüche

1. Verfahren zum Korrigieren einer Position eines Messpunktes in einem Messbild, insbesondere in einem endoskopischen und/oder exoskopischen und/oder mikroskopischen Messbild, zumindest die Schritte umfassend:
- Erfassen eines ersten Messbildes (134) zumindest eines beweglichen Messobjektes (112);
- Bestimmen des zumindest einen Messpunktes (142, 144) in Bezug auf das zumindest eine bewegliche Messobjekt (112) in dem ersten Messbild (134);
- Erfassen zumindest eines zeitlich auf das erste Messbild (134) folgenden, zweiten Messbildes (148) des zumindest einen beweglichen Messobjektes (112);
- Berechnen eines Positionsverschiebungsvektors (150) zwischen dem zumindest einen Messpunkt (142, 144) in dem ersten Messbild (134) und einem zu dem zumindest einen Messpunkt (142, 144) korrespondierenden Bildpunkt in dem zweiten Messbild (148); und
- Korrigieren einer Position des in Bezug auf das erste Messbild (134) bestimmten zumindest einen Messpunktes (142, 144) in dem zweiten Messbild (148) auf Basis des berechneten Positionsverschiebungsvektors (150),
**dadurch gekennzeichnet, dass** das Bestimmen des zumindest einen Messpunktes (142, 144) in Bezug auf das zumindest eine bewegliche Messobjekt (112) auf Basis einer Benutzereingabe, nämlich durch Bewegen eines Cursors (138) innerhalb des ersten Messbildes (134) erfolgt, und das Korrigieren der Position des zumindest einen Messpunktes (142, 144) eine Überlagerung eines Bewegungsvektors (152) des Cursors (138) zum Bestimmen des Messpunktes (142, 144) mit dem Positionsverschiebungsvektor (150) umfasst.

2. Verfahren nach einem der vorstehenden Ansprüche, **dadurch gekennzeichnet, dass** eine Positionsänderung zwischen dem in Bezug auf das erste Messbild (134) bestimmten zumindest einen Messpunkt (142, 144) und dem zu diesem korrespondierenden Bildpunkt in dem zweiten Messbild (148) auf einer Bewegung des beweglichen Messobjektes (112) basiert.

3. Verfahren nach einem der vorstehenden Ansprüche **dadurch gekennzeichnet, dass** das Berechnen des Positionsverschiebungsvektors (150) auf Basis eines Optical-Flow-Algorithmus und/oder eines elastischen Bildregistrierungsalgorithmus und/oder eines Punktewolkenregistrierungsalgorithmus und/oder eines landmarkenbasierten Trackings erfolgt.

4. Verfahren nach einem der vorstehenden Ansprüche, **dadurch gekennzeichnet, dass** das erste und das zweite Messbild (134, 148) jeweils ein Einzelbild einer Stereovideosequenz ist.

5. Verfahren nach einem der vorstehenden Ansprüche, **dadurch gekennzeichnet, dass** der zumindest eine Messpunkt (142, 144) auf einer Benutzerausgabevorrichtung (132), insbesondere stets, in seiner korrigierten Position dargestellt wird.

6. Verfahren zum Ausgeben zumindest einer statistischen Kenngröße zu einem beweglichen Messobjekt (112), das insbesondere in einem endoskopischen und/oder exoskopischen und/oder mikroskopischen Messbild umfasst ist, wobei das Verfahren zumindest die Schritte umfasst:
- Bereitstellen einer Vielzahl von zeitlich aufeinanderfolgenden Messbildern des zumindest einen beweglichen Messobjektes (112), wobei die Vielzahl von Messbildern (134, 148) zumindest eine in Bezug auf das bewegliche Messobjekt (112) auszugebende Messstrecke (146) umfasst, die zwischen einem ersten Messpunkt (142), der in Bezug auf das zumindest eine bewegliche Messobjekt (112) bestimmt wird, und einem zweiten Messpunkt (144), der in Bezug auf das zumindest eine bewegliche Messobjekt (112) bestimmt wird, berechnet wird;
- Ermitteln der zumindest einen statistischen Kenngröße in der Vielzahl von zeitlich aufeinanderfolgenden Messbildern (134, 148), die zumindest teilweise in Zusammenhang mit einer periodischen und/oder frequenzbasierten Bewegung des zumindest einen beweglichen Messobjektes (112) steht, durch statistische Auswertung;
- Ausgeben der zumindest einen statistischen Kenngröße, durch die die Bewegung des zumindest einen beweglichen Messobjektes (112) zumindest teilweise beschrieben wird,
**dadurch gekennzeichnet, dass** eine Positionsänderung des ersten Messpunktes (142) und/oder des zweiten Messpunktes (144) in der Vielzahl von zeitlich aufeinanderfolgenden Messbildern, die zumindest teilweise durch eine Bewegung des zumindest einen beweglichen Messobjektes (112) hervorgerufen wird, durch das Verfahren nach einem der Ansprüche 1 bis 5 korrigiert wird.

7. Verfahren nach Anspruch 6, **dadurch gekennzeichnet, dass** die zumindest eine statistische Kenngröße zumindest einen von einer Frequenz, einer Amplitude, einem Minimalwert, einem Maximalwert, einem Mittelwert, einer Standardabweichung und einem statistischen Fehlerindikator umfasst.

8. Verfahren nach Anspruch 6 oder 7, **dadurch gekennzeichnet, dass** zu der zumindest einen statistischen Kenngröße ein mittlerer, resultierender Messfehler ausgegeben wird.

9. Verfahren nach einem der vorstehenden Ansprüche, **dadurch gekennzeichnet, dass** das erste Messbild (134) und/oder das zweite Messbild (148) und/oder die Vielzahl von Messbildern durch Stereorekonstruktion eines jeweiligen Stereobildpaares auf Basis von optischen und/oder dimensionellen Parametern der Bilderfassungsvorrichtung (104) verarbeitet werden.

10. Messvorrichtung (100), die aufweist:
eine Bilderfassungsvorrichtung (104) mit zumindest einer ersten und einer von dieser beabstandeten zweiten Bilderfassungseinheit (108, 110), eine Auswerteeinrichtung (106), und
einen Prozessor, der dazu eingerichtet ist, die Schritte des Verfahrens nach einem der Ansprüche 1 bis 5 und/oder des Verfahrens nach einem der Ansprüche 6 bis 9 auszuführen.

11. Messvorrichtung nach Anspruch 10, **dadurch gekennzeichnet, dass** die Messvorrichtung (100) ein Stereoendoskop (104) oder ein Stereoexoskop oder ein Stereomikroskop oder ein Laryngoskop umfasst.

12. Messvorrichtung nach Anspruch 10 oder 11, **dadurch gekennzeichnet, dass** sie eine Benutzereingabevorrichtung und/oder eine Benutzerausgabevorrichtung (132) umfasst.

## Claims

1. A method for correcting a position of a measurement point in a measurement image, in particular in an endoscopic and/or exoscopic and/or microscopic measurement image, comprising at least the steps of:
- Capturing a first measurement image (134) of at least one movable measurement object (112);
- Determining the at least one measurement point (142, 144) in relation to the at least one movable measurement object (112) in the first measurement image (134);
- Capturing at least one second measurement image (148), following the first measurement image (134) in time, of the at least one movable measurement object (112);
- Calculating a position displacement vector (150) between the at least one measurement point (142, 144) in the first measurement image (134) and an image point, which corresponds to the at least one measurement point (142, 144), in the second measurement image (148); and
- Correcting a position of the at least one measurement point (142, 144), which is determined in relation to the first measurement image (134), in the second measurement image (148) on the basis of the calculated position displacement vector (150),
**characterised in that**
determining the at least one measurement point (142, 144) in relation to the at least one movable measurement object (112) is carried out on the basis of a user input, namely by moving a cursor (138) within the first measurement image (134), and correcting the position of the at least one measurement point (142, 144) comprises a superposition of a movement vector (152) of the cursor (138) for determining the measurement point (142, 144) with the position displacement vector (150).

2. The method according to one of the preceding claims, **characterised in that** a position change between the at least one measurement point (142, 144), which is determined in relation to the first measurement image (134), and the image point, which corresponds thereto, in the second measurement image (148) is based on a movement of the movable measurement object (112).

3. The method according to one of the preceding claims, **characterised in that** calculating the position displacement vector (150) is carried out on the basis of an optical flow algorithm and/or an elastic image registration algorithm and/or a point cloud registration algorithm and/or a landmark-based tracking.

4. The method according to one of the preceding claims, **characterised in that** the first and second measurement images (134, 148) are each a single image of a stereovideo sequence.

5. The method according to one of the preceding claims, **characterised in that** the at least one measurement point (142, 144) is represented on a user output device (132), in particular always, in its corrected position.

6. A method for outputting at least one statistical characteristic variable relating to a movable measurement object (112), which is included in particular in an endoscopic and/or exoscopic and/or microscopic measurement image, wherein the method comprises at least the steps of:
- Providing a plurality of temporally successive measurement images of the at least one movable measurement object (112), wherein the plurality of measurement images (134, 148) comprises at least one measurement path (146) which is to be output in relation to the movable measurement object (112) and which is calculated between a first measurement point (142), which is determined in relation to the at least one movable measurement object (112), and a second measurement point (144), which is determined in relation to the at least one movable measurement object (112);
- Determining the at least one statistical characteristic variable in the plurality of temporally successive measurement images (134, 148), which is at least partially associated with a periodic and/or frequency-based movement of the at least one movable measurement object (112), by way of statistical evaluation;
- Outputting the at least one statistical characteristic variable by way of which the movement of the at least one movable measurement object (112) is at least partially described,
**characterised in that** a position change of the first measurement point (142) and/or of the second measurement point (144) in the plurality of temporally successive measurement images, which is caused at least partially by a movement of the at least one movable measurement object (112), is corrected by the method according to one of claims 1 to 5.

7. The method according to claim 6, **characterised in that** the at least one statistical characteristic variable comprises at least one of a frequency, an amplitude, a minimum value, a maximum value, a mean value, a standard deviation and a statistical error indicator.

8. The method according to claim 6 or 7, **characterised in that** a mean, resulting measurement error is output for the at least one statistical characteristic variable.

9. The method according to one of the preceding claims, **characterised in that** the first measurement image (134) and/or the second measurement image (148) and/or the plurality of measurement images are processed by stereo reconstruction of a respective stereo image pair on the basis of optical and/or dimensional parameters of the image capture device (104).

10. A measuring device (100) comprising: an image capture device (104) having at least one first and one second image capture unit (108, 110), spaced apart therefrom, an evaluation device (106), and
a processor configured to perform the steps of the method according to one of claims 1 to 5 and/or the method according to one of claims 6 to 9.

11. The measuring device according to claim 10, **characterised in that** the measuring device (100) comprises a stereoendoscope (104) or a stereoexoscope or a stereomicroscope or a laryngoscope.

12. The measuring device according to claim 10 or 11, **characterised in that** it comprises a user input device and/or a user output device (132).

## Revendications

1. Procédé pour corriger une position d'un point de mesure dans une image de mesure, en particulier dans une image de mesure endoscopique et/ou exoscopique et/ou microscopique, comprenant au moins les étapes suivantes :
- acquisition d'une première image de mesure (134) d'au moins un objet de mesure mobile (112) ;
- détermination de l'au moins un point de mesure (142, 144) par rapport à l'au moins un objet de mesure mobile (112) dans la première image de mesure (134) ;
- acquisition d'au moins une seconde image de mesure (148), suivant dans le temps la première image de mesure (134), de l'au moins un objet de mesure mobile (112) ;
- calcul d'un vecteur de décalage de position (150) entre l'au moins un point de mesure (142, 144) dans la première image de mesure (134) et un point d'image correspondant à l'au moins un point de mesure (142, 144) dans la seconde image de mesure (148) ; et
- correction d'une position de l'au moins un point de mesure (142, 144) déterminé par rapport à la première image de mesure (134) dans la seconde image de mesure (148) sur la base du vecteur de décalage de position (150) calculé,
**caractérisé en ce que**
la détermination de l'au moins un point de mesure (142, 144) par rapport à l'au moins un objet de mesure mobile (112) est effectuée sur la base d'une entrée utilisateur, à savoir par le mouvement d'un curseur (138) à l'intérieur de la première image de mesure (134), et la correction de la position de l'au moins un point de mesure (142, 144) comprend une superposition d'un vecteur de mouvement (152) du curseur (138) pour déterminer le point de mesure (142, 144) avec le vecteur de décalage de position (150).

2. Procédé selon l'une des revendications précédentes, **caractérisé en ce qu'**un changement de position entre l'au moins un point de mesure (142, 144) déterminé par rapport à la première image de mesure (134) et le point d'image correspondant à celui-ci dans la seconde image de mesure (148) est basé sur un mouvement de l'objet de mesure mobile (112).

3. Procédé selon l'une des revendications précédentes, **caractérisé en ce que** le calcul du vecteur de décalage de position (150) est effectué sur la base d'un algorithme de flux optique et/ou d'un algorithme d'enregistrement d'image élastique et/ou d'un algorithme d'enregistrement de nuage de points et/ou d'un suivi basé sur des repères.

4. Procédé selon l'une des revendications précédentes, **caractérisé en ce que** la première et la seconde image de mesure (134, 148) sont respectivement une image unique d'une séquence vidéo stéréoscopique.

5. Procédé selon l'une des revendications précédentes, **caractérisé en ce que** l'au moins un point de mesure (142, 144) est représenté sur un dispositif de sortie utilisateur (132), en particulier en permanence, dans sa position corrigée.

6. Procédé pour délivrer au moins une caractéristique statistique à un objet de mesure mobile (112), qui est en particulier compris dans une image de mesure endoscopique et/ou exoscopique et/ou microscopique, dans lequel le procédé comprend au moins les étapes :
- fourniture d'une pluralité d'images de mesure se suivant dans le temps de l'au moins un objet de mesure mobile (112), dans lequel la pluralité d'images de mesure (134, 148) comprend au moins un trajet de mesure (146) à délivrer par rapport à l'objet de mesure mobile (112), qui est calculé entre un premier point de mesure (142), qui est déterminé par rapport à l'au moins un objet de mesure mobile (112) et un second point de mesure (144), qui est déterminé par rapport à l'au moins un objet de mesure mobile (112) ;
- détermination de l'au moins une caractéristique statistique dans la pluralité d'images de mesure (134, 148) se suivant dans le temps, qui est au moins partiellement en relation avec un mouvement périodique et/ou basé sur la fréquence de l'au moins un objet de mesure mobile (112), par évaluation statistique ;
- délivrance de l'au moins une caractéristique statistique par laquelle le mouvement de l'au moins un objet de mesure mobile (112) est au moins partiellement décrit,
**caractérisé en ce qu'**un changement de position du premier point de mesure (142) et/ou du second point de mesure (144) dans la pluralité d'images de mesure se suivant dans le temps, qui est provoqué au moins partiellement par un mouvement de l'au moins un objet de mesure mobile (112), est corrigé par le procédé selon l'une des revendications 1 à 5.

7. Procédé selon la revendication 6, **caractérisé en ce que** l'au moins une caractéristique statistique comprend au moins l'un parmi une fréquence, une amplitude, une valeur minimale, une valeur maximale, une valeur moyenne, un écart type et un indicateur d'erreur statistique.

8. Procédé selon la revendication 6 ou 7, **caractérisé en ce qu'**une erreur de mesure moyenne résultante est délivrée pour l'au moins une caractéristique statistique.

9. Procédé selon l'une des revendications précédentes, **caractérisé en ce que** la première image de mesure (134) et/ou la seconde image de mesure (148) et/ou la pluralité d'images de mesure sont traitées par reconstruction stéréo d'une paire d'images stéréo respective sur la base de paramètres optiques et/ou dimensionnels du dispositif d'acquisition d'images (104).

10. Dispositif de mesure (100), qui présente :
un dispositif d'acquisition d'images (104) comportant au moins une première et une seconde unité d'acquisition d'images (108, 110) espacée de celle-ci, un moyen d'évaluation (106), et
un processeur qui est conçu pour exécuter les étapes du procédé selon l'une des revendications 1 à 5 et/ou du procédé selon l'une des revendications 6 à 9.

11. Dispositif de mesure selon la revendication 10, **caractérisé en ce que** le dispositif de mesure (100) comprend un stéréoendoscope (104) ou un stéréoexoscope ou un stéréomicroscope ou un laryngoscope.

12. Dispositif de mesure selon la revendication 10 ou 11, **caractérisé en ce qu'**il comprend un dispositif d'entrée utilisateur et/ou un dispositif de délivrance utilisateur (132).
